# EUROPÄISCHE PATENTSCHRIFT

(11) **EP 0 524 517 B1**
(45) Veröffentlichungstag und Bekanntmachung des Hinweises auf die Patenterteilung: **21.06.1995**
(21) Anmeldenummer: 92111875.8
(22) Anmeldetag: 13.07.1992
(51) Int. Cl.: C07C 259/06, C07C 275/64, A61K 31/16

(54) **Substituierte 3,4-Dihydronaphthaline, diese Verbindungen enthaltende Arzneimittel und Verfahren zur Herstellung dieser Verbindungen**
Substituted 3,4-dihydronaphthalenes, medicament containing them and process for their preparation
3,4-Dihydronaphthalènes substitués, médicaments les contenant et procédé de leur préparation

(30) Priorität: 23.07.1991 DE 4124345
(43) Veröffentlichungstag der Anmeldung: 27.01.1993
(73) Patentinhaber: Grünenthal GmbH, D-52078 Aachen (DE)
(72) Erfinder: Zimmer, Oswald, Dr., W-5160 Düren (DE); Vollenberg, Werner, Dr., W-5190 Stolberg (DE); Schneider, Johannes, Dr., W-5190 Stolberg (DE)

(56) Entgegenhaltungen:
- EP-A- 0 279 263
- EP-A- 0 292 699
- EP-A- 0 408 760
- EP-A- 0 452 908

## Beschreibung

Polyungesättigte höhere Fettsäuren, wie z. B. die Arachidonsäure dienen im Stoffwechsel des Menschen und von Säugetieren als Substrate für die enzymatisch katalysierte Bildung physiologisch bedeutsamer Eicosanoide wie z. B. Prostaglandine und Leukotriene, einer auch unter dem Namen "Slow reacting Substance of Anaphylaxis" (SRS-A) bekannten Substanzklasse. Dabei wird die Prostaglandinbildung durch die Cyclooxygenase (auch als "Prostaglandinsynthetase" bezeichnet), die Leukotrienbildung durch 5-Lipoxygenase katalysiert.

Während die Prostaglandine eine Anzahl erwünschter Wirkungen im Organismus entfalten, ist von den Leukotrienen bzw. der SRS-A, bekannt, daß sie die Entstehung von lebensbedrohlichen Situationen wie z. B. den anaphylaktischen oder auch den septischen Schock, aber auch allergische Reaktionen, Bronchokonstriktionen, Asthma und weitere unerwünschte Effekte bewirken können. Es wird daher seit längerer Zeit von verschiedenen Seiten angestrebt, über chemisch und metabolisch stabile Verbindungen verfügen zu können, die im Organismus die Prostaglandinbildung unbeeinflußt lassen, gleichzeitig aber möglichst selektiv bzw. spezifisch die 5-Lipoxygenase hemmen und so die Bildung der unerwünschten Leukotriene verhindern. Eine in jeder Richtung befriedigende Lösung dieses Problems ist bisher aber noch nicht gelungen.

Die vorliegende Erfindung bezieht sich auf bestimmte substituierte 3,4-Dihydronaphthaline, bei denen es sich um chemisch und metabolisch (bei therapeutischer Anwendung) stabile Verbindungen handelt, die eine spezifische Hemmwirkung gegenüber der 5-Lipoxygenase und überraschenderweise eine schockhemmende Wirkung sowie zum Teil ausgeprägte, die Schock-Mortalitätsrate vermindernde Effekte und darüber hinaus weitere biologisch bzw. pharmazeutisch wertvolle Eigenschaften besitzen.

Diese Verbindungen entsprechen der allgemeinen Formel I 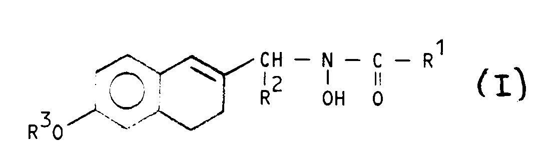
in der R¹ eine Methyl- oder eine Aminogruppe ist, R² ein Wasserstoffatom oder eine Methylgruppe darstellt und R³ einen geradkettigen oder verzweigten Alkylrest mit 1 bis 4 Kohlenstoffatomen, den Allyl- oder den Crotylrest oder eine Cyclopentylgruppe bedeutet.

Verbindungen, in denen R³ den Methylrest bedeutet, werden bevorzugt und solche, in denen R¹ die Aminogruppe bedeutet, besonders bevorzugt.

N-Hydroxy-N-[(6-methoxy-3,4-dihydronaphth-2-yl)methyl]harnstoff ist eine besonders bevorzugte Verbindung, die insbesondere zur Anwendung bei Asthma-Patienten indiziert ist.

Die erfindungsgemäßen substituierten 3,4-Dihydronaphthaline der allgemeinen Formel I besitzen eine spezifische Hemmwirkung gegenüber 5-Lipoxygenase, was u. a. durch invitro-Experimente ermittelt wurde.

Zur Ermittlung der 5-Lipoxygenasehemmung wurden basophile leukämische Leukozyten der Ratte (RBL-1-Zellen) in vitro gezüchtet, vom Nährmedium abzentrifugiert, mit Puffer (Kaliumphosphat 50 mM, pH 7,4) gewaschen und anschließend auf eine Zellzahl von 1 x 10⁷ Zellen pro ml eingestellt. Jeweils 1 ml dieser Zellsuspension wurde mit 10 »M Indomethacin, 2 mM CaCl₂ und entweder mit einer erfindungsgemäßen Verbindung in Konzentrationsbereichen von 0,1 »M bis 100 »M oder mit Lösungsmittel bei Raumtemperatur 3 Minuten vorinkubiert und anschließend mit 20 »M radioaktiv markierter Arachidonsäure und 20 »M des Calcium-Ionophors A 23187 10 Minuten inkubiert. Nach Abstoppen der Reaktion durch Zugabe von 20 »l Eisessig wurden die Reaktionsprodukte mit Ethylacetat extrahiert und mit einem für Lipoxygenaseprodukte geeigneten Laufmittelgemisch dünnschichtchromatographisch getrennt (Jakschik et al., Biochem. Biophys. Res. Commun. 102, 624 (1981)).

Die Radioaktivitätsverteilung der verschiedenen Arachidonsäuremetabolite wurde mit Hilfe eines TLC-Linear-Analyzers gemessen. Aus den prozentualen Bildungsanteilen der 5-Lipoxygenaseprodukte (5-HETE, LTB₄-Isomere) bei Abwesenheit erfindungsgemäßer Verbindungen sowie in Gegenwart verschiedener Konzentrationen an erfindungsgemäßen Verbindungen der Formel I wurden graphisch aus halblogarithmischen Diagrammen "IC₅₀-Werte", d. h. die Konzentrationen, die eine 50%ige Hemmung der 5-Lipoxygenase bewirken, bestimmt. Zur Standardisierung wurden diese Werte auf die im gleichen Test ermittelten IC₅₀-Werte für die Standardsubstanz Nordihydroguaiaretinsäure bezogen. Viele substituierte 3,4-Dihydronaphthaline der Formel I besitzen IC₅₀-Werte von ein Mikromolar oder weniger.

Der Einfluß der erfindungsgemäßen Verbindungen der Formel I auf die Cyclooxygenase-Aktivität wurde mit Hilfe von Schafsamenblasenmikrosomen, suspendiert in Puffer (Kaliumphosphat 50 mM, pH 7,0), durch Inkubation entweder mit einer erfindungsgemäßen Verbindung oder mit Lösungsmittel sowie radioaktiv markierter Arachidonsäure untersucht. Es zeigte sich, daß keine der erfindungsgemäßen Verbindungen in Konzentrationen bis zu 500 Mikromolar die Cyclooxygenase zu hemmen vermochte.

Aus diesen Untersuchungen ergibt sich, daß die IC₅₀-Werte für die Cyclooxygenasehemmung durchweg weit höher als die Werte für die 5-Lipoxygenasehemmung liegen, d. h. daß die erfindungsgemäßen substituierten 3,4-Dihydronaphthaline sehr spezifisch nur die Hemmung der 5-Lipoxygenaseaktivität bewirken.

Zur Überprüfung der in-vivo-Verfügbarkeit der Verbindungen der Formel I (nach oraler Gabe) als 5-Lipoxygenasehemmer wurde ein von Tateson et al., Brit. J. Pharmacol. 94, 528 (1988) beschriebenes Testmodell eingesetzt. Hierzu wurden männlichen Ratten (Stamm Wistar) Verbindungen der Formel I peroral appliziert. Jeweils 1 Stunde nach Substanzapplikation wurde den Tieren nach letaler CO₂-Narkose unter Zusatz von Heparin als Antikoagulans Vollblut entnommen. Aliquote des Vollblutes wurden im Wasserbad bei 37° C 30 Minuten mit dem Calciumionophor A 23187 in einer Endkonzentration von 15 »g/ml inkubiert. Anschließend wurden die Inkubationsansätze zentrifugiert und in aliquoten Teilen des zellfreien Plasmas der Gehalt an immunreaktivem LTB₄ (iLTB₄) per Radio-Immun-Assay "RIA" (3H-LTB₄-RIA, Fa. Amersham) bestimmt. Der iLTB₄-Gehalt jeder Probe wurde anhand einer mit verdünntem Rattenplasma erstellten Standardkurve von LTB₄-Standardkonzentrationen rechnerisch ermittelt und als ng iLTB₄ pro ml Rattenplasma berechnet. Parallel zu den mit einer erfindungsgemäßen Verbindung behandelten Tieren wurden jeweils mit Vehikellösung peroral behandelte Tiere als Kontrollgruppe eingesetzt und ihr Blut untersucht. Der Mittelwert aus den iLTB₄-Gehalten der Rattenplasmen dieser Kontrollgruppen diente als 100%-Wert einer normalen 5-Lipoxygenaseaktivität. Die jeweilige prozentuale Hemmung der ex vivo iLTB₄-Synthese nach oraler Applikation einer erfindungsgemäßen Verbindung wurde wie folgt berechnet: 100 minus dem 100-fachen des Quotienten aus dem Mittelwert der iLTB₄-Gehalte in ng iLTB₄/ml der mit einer erfindungsgemäßen Verbindung behandelten Gruppe und dem Mittelwert der iLTB₄-Gehalte in ng iLTB₄/ml der jeweiligen Kontrollgruppe. Es wurden so beispielsweise ED₅₀-Werte, d. h. die Konzentrationen, die nach oraler Verabreichung an Ratten eine 50 %ige Hemmung der ex vivo iLTB₄-Synthese bewirken, von 13,0 mg/kg, 20,0 mg/kg und 14,7 mg/kg für die nach den Beispielen 1, 2 und 3 hergestellten erfindungsgemäßen Verbindungen ermittelt.

Die antiasthmatische Wirkung erfindungsgemäßer Verbindungen der Formel I wurde an narkotisierten beatmeten Meerschweinchen untersucht. Zur Auslösung einer asthmatischen Reaktion wurden die Tiere passiv mit Antiovalbumin Serum (0,3 ml intraperitoneal) sensibilisiert. Nach 48 Stunden wurde die asthmatische Reaktion durch intravenöse Gabe von 0,2 mg/kg Ovalbumin ausgelöst. Die sofort auftretende Bronchokonstriktion wurde anhand des intra-trachealen Druckanstiegs über einen Zeitraum von 10 Minuten gemessen. Effekte, die durch Histamin, Serotonin und sympathische Gegensteuerung ausgelöst werden, wurden durch intravenöse Vorbehandlung der Tiere mit 2,15 mg/kg Mepyramin, 46,4 mg/kg Propranolol, 4,64 mg/kg Atropin und 1 mg/kg Methysergid (5 Minuten vor Gabe des Ovalbumins) ausgeschaltet. Substituierte 3,4-Dihydronaphthaline der Formel I wurden oral 60 Minuten vor der Ovalbumin-Verabreichung appliziert. Für die Hemmung der durch Ovalbumin ausgelösten Bronchokonstriktionen wurden so beispielsweise ED₄₀-Werte, d. h. effektive Dosen, bei denen die Bronchokonstriktion im Mittel um 40 % gehemmt ist, von 33,5 mg/kg, 27,5 mg/kg und 36,8 mg/kg für die nach den Beispielen 3, 6a und 6e hergestellten Verbindungen ermittelt.

Als ein Testmodell für die Ermittlung der Wirkung der erfindungsgemäßen Verbindungen beim septischen Schock kann die an der Maus durch Endotoxin/Galaktosamin induzierte Hepatitis herangezogen werden. Dabei wurde durch intravenöse Injektion von 300 »g/kg Endotoxin (Lipopolysaccharid von S. abortus equi) zusammen mit 700 mg/kg Galaktosamin an wachen Mäusen eine Hepatitis induziert, die 8 Stunden nach der Endotoxinapplikation durch Anstieg der Aktivitäten leberspezifischer Enzyme (Glutamatpyruvattransaminase GPT; Sorbitoldehydrogenase SDH) nachgewiesen wurde. Die intraperitoneale Applikation erfindungsgemäßer Verbindungen der Formel I 30 Minuten vor, gleichzeitig mit sowie 2, 4 und 6 Stunden nach der Endotoxininjektion hemmte den hepatitisbedingten Anstieg dieser Serumenzymaktivitäten. Bei 5maliger Applikation von je 10 mg/kg erfindungsgemäßer Verbindungen wurden folgende Hemmwerte des Anstiegs der Enzymaktivitäten von GPT und SDH (bezogen auf den Wert 100 % des Anstiegs der Enzymaktivitäten in Kontrolltieren, denen 5-mal nur das Vehikel 1 gew-.-%ige Natriumcarboxymethylcelluloselösung verabreicht worden war) gefunden:

| Beispiel | Hemmung des Anstiegs der Enzymaktivität von | |
|---|---|---|
| | GPT | SDH |
| 1 | 54 % | 41 % |
| 2 | 56 % | 69 % |
| 3 | 78 % | 57 % |

Dabei zeigte sich auch eine Verringerung der Schock-Mortalität durch Verabreichung erfindungsgemäßer Verbindungen der Formel I.

Aus der europäischen Patentanmeldung 0 408 760 A1 sind u. a. Verbindungen bekannt, die der Formel A 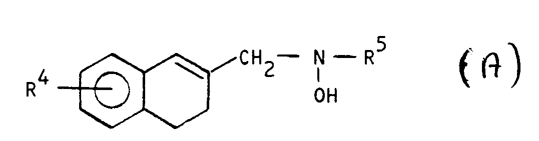
entsprechen, worin R⁴ z. B. ein Aryloxy- oder ein Aryl(nieder)alkoxyrest und R⁵ z. B. der Carbamoyl- oder der Acetylrest sein kann.

Beispielsweise wurden die Verbindungen der Formel A, in denen R⁴ einen Benzyloxyrest in 6-Stellung des 3,4-Dihydronaphthalinrestes darstellt und R⁵ der Carbamoyl- (A₁) oder der Acetylrest (A₂) ist, in dem vorstehend beschriebenen Testmodell der durch Endotoxin/Galaktosamin induzierten Hepatitis eingesetzt. Für die Harnstoffverbindung A₁ konnte weder eine Hemmung von GPT oder von SDH noch eine Verminderung der Schockmortalität beobachtet werden. Die Acetylverbindung A₂ bewirkte nur eine geringe Hemmung von GPT (16 %) und von SDH (32 %) bei nicht feststellbarem Einfluß auf die Schockmortalität.

Aus diesen Vergleichsversuchen ergibt sich der überraschende Befund, daß substituierte 3,4-Dihydronaphthaline der Formel I im Gegensatz zu den strukturverwandten bekannten Verbindungen der Formel A eine ausgeprägte Wirkung beim septischen bzw. anaphylaktischen Schock entfalten.

Die erfindungsgemäßen Verbindungen der Formel I besitzen somit zahlreiche physiologisch wertvolle Eigenschaften wie z. B. antianaphylaktische und antiasthmatische Wirkungen. Da die erfindungsgemäßen Verbindungen der Formel I chemisch und metabolisch für eine therapeutische Anwendung stabil und lagerfähig sind, eignen sie sich zum Einsatz als Arzneimittel, z. B. bei Prophylaxe und Therapie von Schockzuständen, als Antianaphylaktika und als Antiasthmatika.

Die erfindungsgemäßen Verbindungen der Formel I besitzen nur eine geringe Toxizität, die sich erst bei weit höheren als den therapeutisch oder prophylaktisch anzuwendenden Dosierungen bemerkbar macht. Sie können daher als solche in geeigneten pharmazeutischen Zubereitungen an Mensch oder Tier verabreicht werden.

Die Erfindung betrifft dementsprechend auch Arzneimittel, die als Wirkstoff eine oder mehrere der erfindungsgemäßen Verbindungen der Formel I enthalten. Die an den Patienten zu verabreichende Wirkstoffmenge variiert in Abhängigkeit z. B. vom Gewicht des Patienten, vom Applikationsweg, der Indikation und dem Schweregrad der Erkrankung. Unter Berücksichtigung dieser Faktoren beträgt der Wirkstoffgehalt pro Einzeldosis im allgemeinen etwa 0,01 - 1000 mg, und zwar bei Zubereitungsformen für die parenterale Applikation 0,1 bis 1000 mg, bei Zubereitungen für die orale oder rektale Applikation 0,1 bis 1000 mg und bei Zubereitungen für die topische oder inhalative Applikation 0,01 bis 100 mg.

Arzneimittel zur parenteralen Applikation können sowohl Lösungen als auch Suspensionen darstellen, es kommen aber auch leicht rekonstituierbare Trockenzubereitungen in Betracht.

Gut geeignete Applikationsformen sind auch Sprays zur intranasalen oder oralen Applikation bzw. zur Verabreichung der Substanzen über die Bronchien.

Für viele prophylaktische oder therapeutische Anwendungen kommen zweckmäßig auch oral anwendbare Zubereitungsformen der Verbindungen der Formel I, wie Tabletten, Dragees, Kapseln, Granulate, Tropfen und Säfte bzw. Sirupe, aber auch Suppositorien sowie perkutane Applikationszubereitungen, beispielsweise Wirkstoffe in einem Depot in gelöster Form oder gegebenenfalls unter Zusatz von die Hautpenetration fördernden Mitteln enthaltende Pflaster, in Betracht. Vorteilhaft werden diese oral, rektal oder perkutan anwendbaren Zubereitungsformen so hergestellt, daß daraus der Wirkstoff verzögert freigesetzt wird, um so über einen längeren Zeitraum (beispielsweise 24 Stunden) eine gleichmäßige Versorgung des Patienten mit dem Wirkstoff zu gewährleisten.

Alle vorstehend erwähnten pharmazeutischen Zubereitungsformen sind an sich bekannt, und da die erfindungsgemäßen Verbindungen der Formel I chemisch stabil sind, wirft ihre Einarbeitung in diese Zubereitungsformen für den Fachmann keinerlei Probleme auf. Bei der erfindungsgemäßen Herstellung dieser Arzneimittel muß selbstverständlich mit der üblichen Sorgfalt bei Auswahl der Hilfsstoffe wie Trägermaterialien, Lösungsmittel, Verdünnungsmittel, Farbstoffe, Geschmackskorrigentien, Bindemittel, Tablettensprengstoffe, vorgegangen werden, und insbesondere bei der Herstellung von parenteral anzuwendenden Zubereitungsformen ist auf Sterilität und - wenn sie in flüssiger Form vorliegen - Isotonie zu achten.

Zur Herstellung der Verbindungen der allgemeinen Formel I setzt man erfindungsgemäß eine Verbindung der Formel II 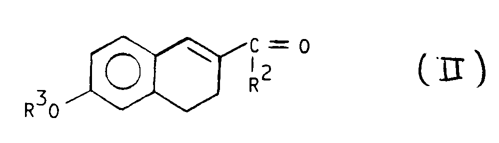
in der R² und R³ die gleiche Bedeutung wie oben haben, mit Hydroxylamin oder einem von dessen Salzen in Gegenwart von Basen zum Oxim um, reduziert dieses mit einem borhaltigen Reduktionsmittel in Gegenwart von Säuren zu einem Hydroxylamin der Formel III 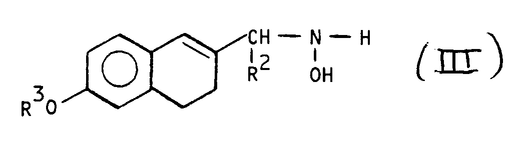
und führt anschließend den Rest -CO-R¹ ein.

Die Umsetzung der Verbindung der Formel II zu ihrem Oxim erfolgt in an sich bekannter Weise, z. B. in alkoholischer oder wäßrig-alkoholischer Lösung in Gegenwart von Basen, z. B. Pyridin, Kaliumcarbonat oder Natriumacetat bei Temperaturen von 20 - 60° C.

Die Reduktion des Oxims wird vorzugsweise mit Borhydriden, insbesondere mit Natriumcyanoborhydrid, in Essigsäure oder ethanolischer Salzsäure bei Temperaturen zwischen 20° C und 60° C oder in alkoholischer Lösung mit Boran-Amin-Komplexen, beispielsweise Boran-Trimethylamin-Komplex oder Boran-Pyridin-Komplex, oder mit Boran-Tetrahydrofuran-Komplex, jeweils in Gegenwart von z. B. 6n Salzsäure bei Temperaturen zwischen 0° C und 50° C durchgeführt (vgl. u. a. J. B. Summers et al., J. Med. Chem. 31, 1960 (1988)).

Durch Einführung des Restes -CO-R¹ erhält man dann aus dem Hydroxylamin der Formel III ein substituiertes 3,4-Dihydronaphthalin der Formel I.

Zur Herstellung von Verbindungen der Formel I mit R¹ = NH₂ (N-Hydroxyharnstoffe) wird ein Hydroxylamin der Formel III zunächst mit Trimethylsilylisocyanat in inerten Lösungsmitteln, vorzugsweise cyclischen Ethern, wie Tetrahydrofuran oder 1,4-Dioxan, auf Temperaturen zwischen 20° C und der Siedetemperatur des Lösungsmittels erwärmt. Durch Hydrolyse des intermediär gebildeten Additionsproduktes mit z. B. gesättigter Ammonium- oder Natriumchloridlösung erhält man die gewünschte Verbindung der Formel I mit R¹ = NH₂.

Alternativ kann man N-Hydroxyharnstoffe (R¹ = NH₂) in an sich bekannter Weise durch Umsetzung eines Hydroxylamins der Formel III entweder mit Kalium- oder Natriumcyanat in saurer Lösung oder mit Phosgen oder einem Chlorameisensäure-(niedrig-alkyl)- oder -benzylester in Gegenwart eines säurebindenden Mittels, wie z. B. Natrium- oder Kaliumcarbonat, gefolgt von einer Behandlung mit Ammoniak oder einer Ammoniak liefernden Verbindung, z. B. Ammoniumcarbonat erhalten.

Zur Herstellung von Verbindungen der Formel I mit R¹ = CH₃ (Acetohydroxamsäuren) wird ein Hydroxylamin der Formel III, gegebenenfalls ohne vorherige Isolierung, zunächst mit einem Acetylierungsmittel, vorzugsweise Acetanhydrid oder Acetylchlorid, in Gegenwart säurebindender Stoffe, wie Pyridin oder Chinolin umgesetzt, wobei eine Verbindung der Formel IV 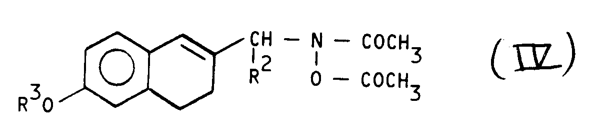
erhalten wird, aus der mit einer Base in einem Lösungsmittel, beispielsweise Methanol oder Ethanol, bei Temperaturen zwischen etwa 20° C und 60° C die O-Acetylgruppe unter Bildung der gewünschten Verbindung der Formel I abgespalten wird. Als Base eignet sich z. B. Kalium-, Natrium- oder Lithiumhydroxid oder Natrium- oder Kaliumcarbonat, die gegebenenfalls in Form einer wäßrigen 0,1 bis 1 normalen Lösung oder alkoholischen Lösung einer Verbindung der Formel IV zugesetzt wird.

Die Herstellung der Ausgangsverbindungen der Formel II erfolgt in an sich bekannter Weise, indem man ein 1-Tetralonderivat der allgemeinen Formel V 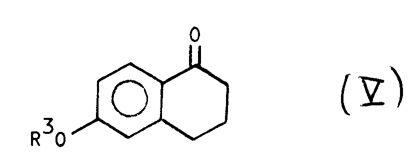
mit einem komplexen Metallhydrid, wie z. B. Natriumborhydrid in Methanol oder Ethanol oder Lithiumaluminiumhydrid in Diethylether, Tetrahydrofuran oder einem anderen geeigneten Ether zu einem 1,2,3,4-Tetrahydro-1-naphtholderivat der Formel VI 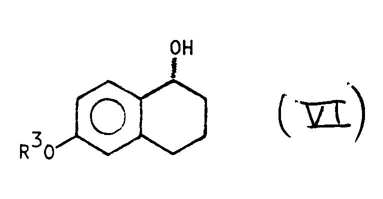
reduziert.

Durch Vilsmeyer-Formylierung mit z. B. Phosphoroxychlorid/Dimethylformamid oder N-Methylformanilid bei Temperaturen zwischen 0° und 70° C erhält man aus der Verbindung der Formel VI in hohen Ausbeuten die entsprechende Verbindung der Formel II, in der R² für ein Wasserstoffatom steht.

Aus der Verbindung der Formel II mit R² = H läßt sich durch Umsetzung mit Methylmagnesiumjodid oder -bromid in Diethylether und anschließende Oxidation des erhaltenen sekundären Alkohols mit z. B. 2,3-Dichlor-5,6-dicyan-p-benzochinon in Benzol oder Toluol bei 20° C die entsprechende Verbindung der Formel II, in der R² für eine Methylgruppe steht, erhalten.

### Beispiele

Alle Temperaturen sind unkorrigiert.

Die ¹H-NMR-Spektren wurden bei 300 MHz gemessen.

Es wurde Petrolether mit einem Siedebereich von 50 - 70° C verwendet.

Als stationäre Phase für die Säulenchromatographie wurde Kieselgel 60 (0,040 - 0,063 mm) der Firma E. Merck, Darmstadt, eingesetzt.

Die dünnschichtchromatographischen Untersuchungen wurden mit "HPTLC Fertigplatten, Kieselgel 60 F 254" der Firma E. Merck, Darmstadt, durchgeführt. Soweit der Reaktionsverlauf dünnschichtchromatographisch kontrolliert wurde, wird in den Beispielen für diese "DC-Kontrolle" jeweils das Laufmittel genannt.

Die Mischungsverhältnisse der Laufmittel für alle chromatographischen Untersuchungen sind stets in Volumen/Volumen angegeben.

### Beispiel 1

### N-[(6-Methoxy-3,4-dihydronaphth-2-yl)methyl]acetohydroxamsäure

### a) 1-Hydroxy-6-methoxy-1,2,3,4-tetrahydronaphthalin

Eine Lösung von 10,68 g 6-Methoxy-1-tetralon in 180 ml Methanol wurde unter Rühren portionsweise mit 4,56 g Natriumborhydrid versetzt, so daß die Temperatur 30° C nicht überstieg. Es wurde noch 1 Stunde bei 20° C gerührt, anschließend auf ein Volumen von ca. 30 ml eingedampft, mit 200 ml Wasser verdünnt und viermal mit je 50 ml Essigsäureethylester extrahiert. Die Extrakte wurden mit gesättigter Natriumchloridlösung gewaschen und über Natriumsulfat getrocknet. Nach destillativer Entfernung des Lösungsmittels wurden 10,34 g (96,2 % d. Th.) 1-Hydroxy-6-methoxy-1,2,3,4-tetrahydronaphthalin in Form eines Öls erhalten.
¹H-NMR (CDCl₃): 1,68 - 2,03 (m, 4H, CH₂);
2,63 - 2,87 (m, 2H, CH₂-aromat.); 3,78 (s, 3H, OCH₃); 4,70 - 4,78 (m, 1H, OCH); 6,61 - 6,62 (d, 1H, aromat.); 6,74 - 6,78 (d,d, 1H, aromat.); 7,31 - 7,34 (d, 1H, aromat.)

### b) 6-Methoxy-3,4-dihydronaphth-2-aldehyd

Eine Lösung von 10,0 g der nach Beispiel 1a hergestellten Verbindung in 35 ml Dimethylformamid wurde bei 0 - 5° C unter Rühren und Überleiten von trockenem Stickstoff tropfenweise mit einer frisch bereiteten Mischung aus 8,3 ml Phosphoroxychlorid und 11,6 ml Dimethylformamid versetzt. Danach wurde innerhalb einer Stunde auf 75 - 80° C erwärmt und bei dieser Temperatur bis zur vollständigen Umsetzung gerührt (DC-Kontrolle: Petrolether/Diethylether - 1 : 1). Es wurde auf Raumtemperatur abgekühlt, eine Lösung von 36,6 g Natriumacetat in 85 ml Wasser zugetropft und dann mit Essigsäureethylester extrahiert. Die Extrakte wurden mit gesättigter Natriumchloridlösung gewaschen und über Natriumsulfat getrocknet. Nach Chromatographie mit Petrolether/Diethylether (1 : 1) wurden 9,92 g (94,5 % d. Th.) des Aldehydes in Form gelb gefärbter Kristalle mit einem Schmelzpunkt von 48 - 49° C erhalten.
¹H-NMR (CDCl₃): 2,51 - 2,57 (m, 2H, CH₂);
2,82 - 2,87 (t, 2H, CH₂); 3,83 (s, 3H, OCH₃); 6,75 - 6,79 (m, 2H, aromat.); 7,21 - 7,23 (m, 1H, aromat.); 7,24 (s, 1H, olefin.); 9,60 (s, 1H, CHO)

### c) 6-Methoxy-3,4-dihydronaphth-2-aldehyd-oxim

Eine Lösung von 9,88 g des nach Beispiel 1b hergestellten Aldehydes, 8,61 g Natriumacetat und 9,18 g Hydroxylamin-Hydrochlorid in einem Gemisch aus je 75 ml Methanol und Tetrahydrofuran sowie 90 ml Wasser wurde 8 Stunden bei 60° C Badtemperatur gerührt. Dann wurde eingedampft, der Rückstand in 200 ml Essigsäureethylester aufgenommen und die Lösung mit Wasser und gesättigter Natriumchloridlösung gewaschen. Nach Trocknen über Natriumsulfat, Eindampfen im Vakuum und Umkristallisieren aus Diethylether/n-Hexan wurden 9,45 g (88,6 % d. Th.) des Oxims in Form gelb gefärbter Kristalle mit einem Schmelzpunkt von 169 - 172° C erhalten.
¹H-NMR (CDCl₃): 2,56 - 2,61 (t, 2H, CH₂);
2,82 - 2,88 (t, 2H, CH₂); 3,82 (s, 3H, OCH₃); 6,61 (s, 1H, olefin.); 6,70 - 6,73 (m, 2H, aromat.); 7,04 - 7,07 (m, 1H, aromat.); 7,89 (s, 1H, N=CH)

### d) N-[(6-Methoxy-3,4-dihydronaphth-2-yl)methyl]acetohydroxamsäure

Eine Lösung von 9,35 g des nach Beispiel 1c hergestellten Oxims in 100 ml Essigsäure wurde bei 50 - 55° C Badtemperatur unter Rühren und Überleiten von trockenem Stickstoff portionsweise mit 4,35 g Natriumcyanoborhydrid versetzt. Es wurde noch eine Stunde bei dieser Temperatur gerührt, dann auf 20° C abgekühlt, mit 8,75 ml Acetanhydrid versetzt und zwölf Stunden gerührt. Dann wurde im Vakuum eingedampft, der Rückstand mit 150 ml Wasser verdünnt und dreimal mit je 50 ml Essigsäureethylester extrahiert. Die Extrakte wurden mit gesättigten wäßrigen Lösungen von Natriumhydrogencarbonat bzw. Natriumchlorid gewaschen und über Natriumsulfat getrocknet. Aus dem Eindampfrückstand wurde durch Chromatographie mit Essigsäureethylester/Petrolether (2 : 1) die entstandene Verbindung der Formel IV isoliert. Zu dieser Verbindung, gelöst in 100 ml Methanol, wurden 100 ml 10 gew.-%ige wäßrige Natriumcarbonatlösung gegeben. Das Gemisch wurde dann unter Rühren zwei Stunden auf 60° C erwärmt. Es wurde im Vakuum eingedampft und der Rückstand aus Diethylether/n-Hexan umkristallisiert, wobei 8,10 g (71,3 % d. Th.) der Acetohydroxamsäure in Form farbloser Kristalle mit einem Schmelzpunkt von 141 - 142° C erhalten wurden.
¹H-NMR (DMSO-d₆): 2,05 (s, 3H, COCH₃);
2,10 - 2,16 (m, 2H, CH₂); 2,71 - 2,77 (m, 2H, CH₂); 3,73 (s, 3H, OCH₃); 4,22 (s, 2H, NCH₂); 6,28 (s, 1H, olefin.); 6,67 - 6,71 (m, 2H, aromat.); 6,95, 6,98 (d, 1H, aromat.)

### Beispiel 2

### N-[1-(6-Methoxy-3,4-dihydronaphth-2-yl)ethyl]acetohydroxamsäure

### a) 2-(1-Hydroxyethyl)-6-methoxy-3,4-dihydronaphthalin

Zu einer Grignard-Lösung aus 2,93 g Magnesiumspänen und 7,5 ml Methyljodid in 60 ml absolutem Diethylether wurde bei 0 - 5° C unter Rühren tropfenweise eine Lösung von 18,82 g des nach Beispiel 1b hergestellten Aldehydes in 100 ml absolutem Diethylether gegeben. Es wurde bis zur Vervollständigung der Reaktion bei 5° C gerührt (DC-Kontrolle: Petrolether/Diethylether - 1:2) und dann durch Zutropfen von 100 ml gesättigter Ammoniumchloridlösung hydrolysiert. Die organische Phase wurde abgetrennt und die wäßrige zweimal mit je 50 ml Diethylether extrahiert. Die vereinigten organischen Extrakte wurden mit gesättigter Natriumchloridlösung gewaschen und über Natriumsulfat getrocknet. Nach Chromatographie mit Petrolether/Diethylether (1 : 1) wurden 18,29 g (89,5 % d. Th.) des Dihydronaphthalins in Form gelb gefärbter Kristalle mit einem Schmelzpunkt von 32 - 34° C erhalten.
¹H-NMR (CDCl₃): 1,34 - 1,36 (d, 3H, CH₃);
2,19 - 2,40 (m, 2H, CH₂); 2,78 - 2,83 (t, 2H, CH₂); 3,80 (s, 3H, OCH₃); 4,40 - 4,46 (q, 1H, OCH); 6,39 (s, 1H, olefin.); 6,67 - 6,70 (m, 2H, aromat.); 6,96 - 6,99 (m, 1H, aromat.)

### b) 2-Acetyl-6-methoxy-3,4-dihydronaphthalin

Eine Lösung von 18,19 g des nach Beispiel 2a hergestellten Dihydronaphthalins in 450 ml wasserfreiem Toluol wurde bei 20° C unter Rühren und Überleiten von trockenem Stickstoff portionsweise mit 20,6 g 2,3-Dichlor-5,6-dicyan-p-benzochinon versetzt (DC-Kontrolle: Petrolether/Diethylether - 1 : 1). Nach beendeter Umsetzung wurde filtriert, mit Toluol gewaschen und das Filtrat eingedampft. Der Rückstand wurde durch Chromatographie mit Petrolether/Diethylether (1 : 1) gereinigt. Es wurden 15,36 g (85,3 % d. Th.) 2-Acetyl-6-methoxy-3,4-dihydronaphthalin in Form leicht gelb gefärbter Kristalle mit einem Schmelzpunkt von 70 - 72° C erhalten.
¹H-NMR (CDCl₃): 2,42 (s, 3H, COCH₃);
2,54 - 2,57 (m, 2H, CH₂); 2,79 - 2,84 (m, 2H, CH₂); 3,83 (s, 3H, OCH₃); 6,74 - 6,78 (m, 2H, aromat.); 7,17, 7,19 (d, 1H, aromat.); 7,38 (s, 1H, olefin.)

### c) Oxim des 2-Acetyl-6-methoxy-3,4-dihydronaphthalins

Analog Beispiel 1c wurden aus 15,27 g der nach Beispiel 2b erhaltenen Verbindung, 12,38 g Natriumacetat und 13,20 g Hydroxylamin-Hydrochlorid 15,39 g (93,8 % d. Th.) des Oxims in Form nahezu farbloser Kristalle mit einem Schmelzpunkt von 160 - 163° C erhalten.
¹H-NMR (CDCl₃): 2,17 (s, 3H, NCCH₃);
2,60 - 2,65 (m, 2H, CH₂); 2,80 - 2,85 (m, 2H, CH₂); 3,81 (s, 3H, OCH₃); 6,71 - 6,73 (m, 2H, aromat.); 6,80 (s, 1H, olefin.); 7,05 - 7,09 (m, 1H, aromat.)

### d) N-[1-(6-Methoxy-3,4-dihydronaphth-2-yl)ethyl]acetohydroxamsäure

15,21 g der nach Beispiel 2c erhaltenen Verbindung wurden analog Beispiel 1d nacheinander mit 6,93 g Natriumcyanoborhydrid, 16,8 ml Acetanhydrid und 10 gew.%iger wäßriger Natriumcarbonatlösung umgesetzt. Nach Umkristallisieren aus Essigsäureethylester/n-Hexan wurden 8,0 g (43,7 % d. Th.) der Acetohydroxamsäure in Form farbloser Kristalle mit einem Schmelzpunkt von 164 - 165° C erhalten .
¹H-NMR (DMSO-d₆): 1,29, 1,31 (d, 3H, CH₃);
2,03 (s, 3H, NCOCH₃); 2,01 - 2,24 (m, 2H, CH₂); 2,69 - 2,74 (m, 2H, CH₂); 3,73 (s, 3H, OCH₃); 5,06 - 5,17 (m, 1H, NCH); 6,29 (s, 1H, olefin.); 6,67 - 6,70 (m, 2H, aromat.); 6,97, 7,00 (d, 1H, aromat.)

### Beispiel 3

### N-Hydroxy-N-[(6-methoxy-3,4-dihydronaphth-2-yl)methyl]harnstoff

10,16 g der nach Beispiel 1c hergestellten Verbindung wurden entsprechend Beispiel 1d in 100 ml Essigsäure mit 4,73 g Natriumcyanoborhydrid versetzt. Sobald die Reaktion beendet war, wurde im Vakuum eingedampft, der Rückstand in 100 ml Essigsäureethylester aufgenommen und die Lösung je zweimal mit gesättigten Lösungen von Natriumhydrogencarbonat und Natriumchlorid gewaschen. Nach Trocknen über Natriumsulfat und Eindampfen wurde ein viskoses Material erhalten, das in 150 ml trockenem 1,4-Dioxan gelöst wurde. Nach Zugabe von 10,5 ml Trimethylsilyl-isocyanat wurde zwei Stunden unter Rückfluß erhitzt. Nach dem Abkühlen wurde mit gesättigten Lösungen von Ammoniumchlorid und Natriumchlorid gewaschen und über Natriumsulfat getrocknet. Der nach Eindampfen erhaltene Rückstand wurde aus Essigsäureethylester umkristallisiert, wobei 6,79 g (54,7 % d. Th.) des Harnstoffs in Form farbloser Kristalle, die bei 139 - 140° C unter Zersetzung schmolzen, erhalten wurden.
¹H-NMR (DMSO-d₆): 2,14 - 2,19 (m, 2H, CH₂);
2,71 - 2,77 (m, 2H, CH₂); 3,73 (s, 3H, OCH₃); 4,06 (s, 2H, NCH₂); 6,29 (s, 1H, olefin.); 6,33 (s, 2H, CONH₂); 6,66 - 6,70 (m, 2H, aromat.); 6,92, 6,95 (d, 1H, aromat.)

### Beispiel 4

### N-[(6-n-Butoxy-3,4-dihydronaphth-2-yl)methyl]acetohydroxamsäure

### a) 6-Hydroxy-1-tetralon

Ein Gemisch aus 50 g 6-Methoxy-1-tetralon, 200 ml Essigsäure und 400 ml Bromwasserstoffsäure (47 % HBr) wurde 24 Stunden unter Rückfluß erhitzt. Nach dem Abkühlen wurde das Reaktionsgemisch in 3 l Wasser gegossen, der Feststoff abfiltriert und das Filtrat dreimal mit Essigsäureethylester extrahiert. Die Extrakte wurden mit wäßriger Natriumcarbonatlösung neutral gewaschen, über Natriumsulfat getrocknet und eingedampft. Der Rückstand wurde zusammen mit dem abfiltrierten Feststoff aus Essigsäureethylester umkristallisiert. Es wurden 40,75 g (89,5 % d. Th.) 6-Hydroxy-1-tetralon in Form rötlich-brauner Kristalle mit einem Schmelzpunkt von 153 - 155° C erhalten.
¹H-NMR (CDCl₃): 2,07 - 2,15 (m, 2H, CH₂);
2,62 - 2,66 (t, 2H, CH₂); 2,89 - 2,93 (t, 2H, CH₂); 6,72, 6,73 (d, 1H, aromat.); 6,80 - 6,83 (d,d, 1H, aromat.); 7,97, 8,00 (d, 1H, aromat.)

### b) 6-n-Butoxy-1-tetralon

Eine Lösung von 2,50 g 6-Hydroxy-1-tetralon in 20 ml Aceton wurde mit 2,54 g Kaliumcarbonat und 2 ml 1-Brombutan vier Tage unter Rückfluß erhitzt. Danach wurde eingedampft, der Rückstand vorsichtig in 20 ml 1 N Salzsäure aufgenommen, dreimal mit Essigsäureethylester extrahiert und die Extrakte mit gesättigten Lösungen von Natriumhydrogencarbonat und Natriumchlorid gewaschen. Nach Trocknen über Natriumsulfat wurde eingedampft und der erhaltene Rückstand durch Chromatographie mit Diethylether/Petrolether (1 : 2) gereinigt. Es wurden 3,08 g (91,5 % d. Th.) 6-n-Butoxy-1-tetralon in Form eines nahezu farblosen Öls erhalten.
¹H-NMR (CDCl₃): 0,96 - 1,01 (t, 3H, CH₃);
1,43 - 1,56 (m, 2H, CH₂); 1,73 - 1,83 (m, 2H, CH₂); 2,07 - 2,15 (m, 2H, CH₂); 2,58 - 2,63 (t, 2H, CH₂); 2,89 - 2,94 (t, 2H, CH₂); 3,99 - 4,03 (t, 2H, OCH₂); 6,69, 6,70 (d, 1H, aromat.); 6,79 - 6,83 (d,d, 1H, aromat.); 7,98, 8,01 (d, 1H, aromat.)

Das gleiche Produkt ließ sich auch aus 6-Hydroxy-1-tetralon unter Verwendung von Kaliumcarbonat und 1-Butyljodid in Dimethylformamid in 88 %iger Ausbeute erhalten.

### c) 6-n-Butoxy-1-hydroxy-1,2,3,4-tetrahydronaphthalin

Eine Lösung von 21,9 g der nach Beispiel 4b erhaltenen Verbindung in 300 ml Ethanol wurde analog Beispiel 1a mit 7,65 g Natriumborhydrid umgesetzt. Nach Chromatographie mit Petrolether/Diethylether (1 : 1) wurden 21,15 g (96,0 % d. Th.) der gewünschten Verbindung erhalten, die langsam erstarrte und dann bei 38 - 40° C schmolz.
¹H-NMR (CDCl₃): 0,94 - 0,99 (t, 3H, CH₃);
1,44 - 1,54 (m, 2H, CH₂); 1,68 - 1,81 (m, 2H, CH₂); 1,85 - 2,02 (m, 2H, CH₂); 2,63, 2,82 (m, 2H, CH₂); 3,92 - 3,96 (t, 2H, OCH₂); 4,70 - 4,76 (m, 1H, OCH); 6,61, 6,62 (d, 1H; aromat.); 6,74 - 6,77 (d,d, 1H, aromat.); 7,30, 7,33 (d, 1H, aromat.)

### d) 6-n-Butoxy-3,4-dihydro-2-naphthaldehyd

21,15 g der nach Beispiel 4c erhaltenen Verbindung wurden analog Beispiel 1b mit 14,3 ml Phosphoroxychlorid und 20,9 ml Dimethylformamid umgesetzt. Nach Chromatographie mit Petrolether/Diethylether (2 : 1) wurden 19,35 g (87,5 % d. Th.) des Naphthaldehyds in Form farbloser Kristalle mit einem Schmelzpunkt von 62 - 64° C erhalten.
¹H-NMR (CDCl₃): 0,96 - 1,01 (t, 3H, CH₃);
1,44 - 1,56 (m, 2H, CH₂); 1,71 - 1,82 (s, 2H, CH₂); 2,52 - 2,57 (m, 2H, CH₂); 2,81 - 2,87 (m, 2H, CH₂); 3,97 - 4,01 (t, 2H, OCH₂); 6,75 - 6,78 (m, 2H, aromat.,olefin.); 7,20 - 7,23 (m, 2H, aromat.); 9,61 (s, 1H, CHO)

### e) 6-n-Butoxy-3,4-dihydro-2-naphthaldehyd-oxim

19,12 g der nach Beispiel 4d erhaltenen Verbindung wurden analog Beispiel 1c mit 8,7 g Hydroxylamin-Hydrochlorid und 8,15 g Natriumacetat zur Reaktion gebracht. Es wurden 18,83 g (92,5 % d. Th.) des Oxims in Form eines weißen Pulvers mit einem Schmelzpunkt von 116 - 118° C erhalten.
¹H-NMR (CDCl₃): 0,95 - 1,00 (t, 3H, CH₃);
1,43 - 1,55 (m, 2H, CH₂); 1,72 - 1,81 (m, 2H, CH₂); 2,55 - 2,60 (m, 2H, CH₂); 2,81 - 2,87 (m, 2H, CH₂); 3,94 - 3,99 (t, 2H, OCH₂); 6,61 (s, 1H, olefin.); 6,68 - 6,71 (m, 2H, aromat.); 7,02, 7,05 (d, 1H, aromat.); 7,89 (s, 1H, N=CH)

### f) N-[(6-n-Butoxy-3,4-dihydronaphth-2-yl)methyl]acetohydroxamsäure

18,4 g der nach Beispiel 4e erhaltenen Verbindung wurden analog Beispiel 1d mit 7,16 g Natriumcyanoborhydrid und 18 ml Acetanhydrid umgesetzt. Die O-acetylierte Acetohydroxamsäure wurde chromatographisch isoliert (Petrolether/Diethylether - 1 : 3) und dann selektiv mit wäßriger Natriumcarbonatlösung verseift. Nach Umkristallisieren aus Essigsäureethylester/n-Hexan wurden 13,72 g (63,2 % d. Th.) der Hydroxamsäure in Form farbloser Kristalle mit einem Schmelzpunkt von 97 - 98° C erhalten.
¹H-NMR (DMSO-d₆): 0,91 - 0,96 (t, 3H, CH₃);
1,39 - 1,49 (m, 2H, CH₂); 1,63 - 1,70 (m, 2H, CH₂); 2,04 (s, 3H, COCH₃); 2,10 - 2,15 (m, 2H, CH₂); 2,70 - 2,76 (m, 2H, CH₂); 3,91 - 3,95 (t, 2H, OCH₂); 4,21 (s, 2H, NCH₂);6,28 (s, 1H, olefin.); 6,65 - 6,70 (m, 2H, aromat.); 6,93, 6,95 (d, 1H, aromat.)

### Beispiel 5

### N-Hydroxy-N-[(6-cyclopentyloxy-3,4-dihydronaphth-2-yl)methyl]harnstoff

### a) 6-Cyclopentyloxy-1-tetralon

13,0 g der nach Beispiel 4a erhaltenen Verbindung und 10,5 ml Cyclopentylchlorid wurden wie in Beispiel 4b beschrieben in 65 ml Aceton in Gegenwart von 13,8 g Kaliumcarbonat zur Reaktion gebracht. Nach Aufarbeitung und Reinigung wurden 14,65 g (79,5 % d. Th.) der gewünschten Verbindung in Form eines gelb gefärbten Öls erhalten.
¹H-NMR (CDCl₃): 1,57 - 2,01 (m, 8H, CH₂);
2,06 - 2,15 (m, 2H, CH₂); 2,57 - 2,62 (m, 2H, CH₂); 2,88 - 2,92 (m, 2H, CH₂); 4,70 - 4,84 (m, 1H, OCH); 6,66, 6,67 (d, 1H, aromat.); 6,76 - 6,80 (d,d, 1H, aromat.); 7,97, 8,00 (d, 1H, aromat.)

### b) 6-Cyclopentyloxy-1-hydroxy-1,2,3,4-tetrahydronaphthalin

Zu einer Lösung von 1,52 g Lithiumaluminiumhydrid in 75 ml absolutem Diethylether wurde unter Rühren eine Lösung von 16,45 g der nach Beispiel 5a erhaltenen Verbindung in 30 ml Diethylether in der Weise gegeben, daß ein schwaches Sieden aufrechterhalten blieb. Nach beendeter Zugabe wurde drei Stunden unter Rückfluß erhitzt. Dann wurde überschüssiges Hydrid durch vorsichtiges Zutropfen von Wasser zersetzt. Die Etherphase wurde abgetrennt und die wäßrige Phase dreimal mit je 20 ml Diethylether ausgeschüttelt. Die vereinigten organischen Phasen wurden mit gesättigter Natriumchloridlösung gewaschen und über Natriumsulfat getrocknet. Nach Eindampfen und chromatographischer Reinigung des Rückstandes mit Petrolether/Diethylether (1 : 1) wurden 12,32 g (74,2 % d. Th.) der gewünschten Verbindung in Form eines rötlich gefärbten Öls erhalten.
¹H-NMR (CDCl₃): 1,52 - 2,02 (m, 12H, CH₂);
2,61 - 2,86 (m, 2H, CH₂); 4,68 - 4,80 (m, 1H, OCH); 6,59, 6,60 (d, 1H, aromat.); 6,71 - 6,75 (d,d, 1H, aromat.); 7,29, 7,32 (d, 1H, aromat.)

### c) 6-Cyclopentyloxy-3,4-dihydronaphth-2-aldehyd

10,2 g der nach Beispiel 5b erhaltenen Verbindung, gelöst in 15 ml 1,2-Dichlorbenzol, wurden zunächst mit 6 ml N-Methylformanilid, dann tropfenweise mit 4,5 ml Phosphoroxychlorid versetzt. Das Gemisch wurde im schwachen Stickstoffstrom bei 65° C Badtemperatur gerührt (DC-Kontrolle: Petrolether/Diethylether - 2 : 1). Nach beendeter Umsetzung wurde bei 20° C eine Lösung von 28 g Natriumacetat in 65 ml Wasser zugetropft und dann dreimal mit je 30 ml Essigsäureethylester extrahiert. Die Extrakte wurden mit gesättigter Natriumchloridlösung gewaschen, über Natriumsulfat getrocknet und eingedampft. Nach chromatographischer Reinigung des Rückstandes mit Petrolether/Diethylether (1 : 1) wurden 7,37 g (69,3 % d. Th.) des Aldehyds in Form eines gelb gefärbten viskosen Materials erhalten.
¹H-NMR (CDCl₃): 1,55 - 2,00 (m, 8H, CH₂);
2,52 - 2,57 (m, 2H, CH₂); 2,81 - 2,86 (m, 2H, CH₂); 4,76 - 4,84 (m, 1H, OCH); 6,72 - 6,75 (m, 2H, aromat., olefin.); 7,19 - 7,23 (m, 2H, aromat.); 9,79 (s, 1H, CHO)

### d) 6-Cyclopentyloxy-3,4-dihydronaphth-2-aldehyd-oxim

Ein Gemisch aus 5,2 g Hydroxylamin-Hydrochlorid, 7,27 g der nach Beispiel 5c erhaltenen Verbindung, 6,38 g Natriumcarbonat und 120 ml Ethanol/Wasser (2 : 1) wurde sechs Stunden bei 50° C gerührt, dann in 250 ml gesättigte Natriumchloridlösung gegeben und dreimal mit je 100 ml Essigsäureethylester extrahiert. Die Extrakte wurden mit gesättigter Natriumchloridlösung gewaschen, über Magnesiumsulfat getrocknet und zur Trockne eingedampft, wobei 7,46 g (96,6 % d. Th.) des Oxims als blaßgelbes Pulver mit einem Schmelzpunkt von 166 - 168° C erhalten wurden.
¹H-NMR (CDCl₃): 1,57 - 1,97 (m, 8H, CH₂);
2,54 - 2,60 (m, 2H, CH₂); 2,80 - 2,86 (m, 2H, CH₂); 4,71 - 4,80 (m, 1H, OCH); 6,60 (s, 1H, olefin.); 6,66 - 6,69 (m, 2H, aromat.); 7,01 - 7,04 (m, 1H, aromat.); 7,89 (s, 1H, N=CH)

### e) N-Hydroxy-N-[(6-cyclopentyloxy-3,4-dihydronaphth-2-yl)methyl]harnstoff

7,20 g der nach Beispiel 5d erhaltenen Verbindung wurden in 140 ml Ethanol bei 0° C mit 5,26 g Boran-Trimethylamin-Komplex versetzt und eine Stunde gerührt. Dann wurden 70 ml 20gew.-%ige Salzsäure zugetropft. Anschließend wurde unter Rühren und DC-Kontrolle (Essigsäureethylester/Methanol - 30 : 1) auf 20° C erwärmt. Nach vollständiger Reduktion zum Hydroxylamin wurde im Vakuum eingeengt, der Rückstand mit Wasser verdünnt und durch Zugabe von Kaliumcarbonat ein pH-Wert von 9 eingestellt. Anschließend wurde dreimal mit Essigsäureethylester extrahiert. Die Extrakte wurden mit gesättigter Natriumchloridlösung gewaschen und über Natriumsulfat getrocknet. Der nach Eindampfen erhaltene Rückstand wurde in 100 ml absolutem Tetrahydrofuran gelöst, mit 3,8 ml Trimethylsilyl-isocyanat versetzt und drei Stunden bei 50° C gerührt. Es wurde mit 50 ml Essigsäureethylester verdünnt, mit gesättigten Lösungen von Ammonium- und Natriumchlorid gewaschen und über Natriumsulfat getrocknet. Der nach Eindampfen erhaltene Rückstand wurde in Diethylether aufgeschlämmt. Der Feststoff wurde abfiltriert, mit Diethylether gewaschen und aus Essigsäureethylester/n-Hexan umkristallisiert. Es wurden 5,31 g (62,7 % d. Th.) des Harnstoffs in Form farbloser Kristalle mit einem Schmelzpunkt von 145 - 147° C erhalten.
¹H-NMR (DMSO-d₆): 1,50 - 1,77 (m, 6H, CH₂);
1,81 - 1,96 (m, 2H, CH₂); 2,13 - 2,18 (m, 2H, CH₂); 2,69 - 2,75 (m, 2H, CH₂); 4,05 (s, 2H, NCH₂); 4,73 - 4,80 (m, 1H, OCH); 6,27 (s, 1H, olefin.); 6,34 (s, 2H, CONH₂); 6,61 - 6,65 (m, 2H, aromat.); 6,89, 6,92 (d, 1H, aromat.)

### Beispiel 6

Unter den in den Beispielen 1 - 5 angegebenen Bedingungen wurden aus den entsprechenden Ausgangsmaterialien folgende Verbindungen erhalten:

### a) N-[(6-Cyclopentyloxy-3,4-dihydronaphth-2-yl]methyl]acetohydroxamsäure

Schmelzpunkt: 110 - 111° C
¹H-NMR (DMSO-d₆): 1,31 - 1,95 (m, 8H, CH₂);
2,04 (s, 3H, COCH₃); 2,10 - 2,15 (t, 2H, CH₂); 2,70 - 2,75 (t, 2H, CH₂);4,21 (s, 2H, NCH₂); 4,74 - 4,80 (m, 1H, OCH); 6,27 (s, 1H, olefin.); 6,62 - 6,66 (m, 2H, aromat.); 6,92, 6,94 (d, 1H, aromat.)

### b) N-Hydroxy-N-[1-(6-methoxy-3,4-dihydronaphth-2-yl)ethyl]harnstoff

Schmelzpunkt: 146 - 148° C (Zers.)
¹H-NMR (DMSO-d₆): 1,23, 1,25 (d, 3H, NCCH₃);
2,06 - 2,32 (m, 2H, CH₂); 2,62 - 2,77 (m, 2H, CH₂); 3,72 (s, 3H, OCH₃); 4,74 - 4,80 (m, 1H, NCH); 6,27 (s, 1H, olefin.); 6,31 (s, 2H, CONH₂); 6,66 - 6,70 (m, 2H, aromat.); 6,95, 6,98 (d, 1H, aromat.)

### c) N-Hydroxy-N-[(6-n-butoxy-3,4-dihydronaphth-2-yl)methyl]harnstoff

Schmelzpunkt: 150 - 152° C (Zers.)
¹H-NMR (DMSO-d₆): 0,91 - 0,95 (t, 3H, CH₃);
1,37 - 1,49 (m, 2H, CH₂); 1,63 - 1,72 (m, 2H, CH₂); 2,13 - 2,19 (t, 2H, CH₂); 2,70 - 2,76 (t, 2H, CH₂); 3,90 - 3,94 (t, 2H, OCH₂); 4,06 (s, 2H, NCH₂); 6,28 (s, 1H, olefin.); 6,34 (s, 2H, CONH₂); 6,64 - 6,69 (m, 2H, aromat.); 6,90, 6,93 (d, 1H, aromat.)

### d) N-Hydroxy-N-[(6-allyloxy-3,4-dihydronaphth-2-yl)-methyl]harnstoff

Schmelzpunkt: 148 - 149° C (Zers.)
¹H-NMR (DMSO-d₆): 2,15 - 2,20 (t, 2H, CH₂);
2,71 - 2,77 (t, 2H, CH₂); 4,07 (s, 2H, NCH₂); 4,51, 4,53 (d, 2H, OCH₂); 5,22 - 5,41 (m, 2H, olefin.); 5,96 - 6,09 (m, 1H, olefin.); 6,29 (s, 1H, olefin.); 6,32 (s, 2H, CONH₂); 6,67 - 6,71 (m, 2H, aromat.); 6,91, 6,93 (d, 1H, aromat.)

### e) N-Hydroxy-N-[(6-isopropoxy-3,4-dihydronaphth-2-yl)methyl]harnstoff

Schmelzpunkt: 136° C (Zers.)
¹H-NMR (DMSO-d₆): 1,23, 1,25 (d, 6H, CH₃);
2,12 - 2,18 (t, 2H, CH₂); 2,69 - 2,75 (t, 2H, CH₂); 4,05 (s, 2H, NCH₂); 4,50 - 4,62 (m, 1H, OCH); 6,28 (s, 1H, olefin.); 6,34 (s, 2H, CONH₂); 6,64 - 6,68 (m, 2H, aromat.); 6,90, 6,93 (d, 1H, aromat.)

## Patentansprüche

1. Substituierte 3,4-Dihydronaphthaline der allgemeinen Formel I 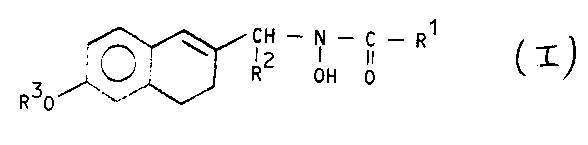 in der R¹ eine Methyl- oder Aminogruppe ist, R² ein Wasserstoffatom oder eine Methylgruppe darstellt und R³ einen geradkettigen oder verzweigten Alkylrest mit 1 bis 4 Kohlenstoffatomen, den Allyl- oder den Crotylrest oder eine Cyclopentylgruppe bedeutet.

2. Substituierte 3,4-Dihydronaphthaline nach Anspruch 1, dadurch gekennzeichnet, daß R³ den Methylrest bedeutet.

3. Substituierte 3,4-Dihydronaphthaline nach einem oder beiden der Ansprüche 1 bis 2, dadurch gekennzeichnet, daß R¹ die Aminogruppe bedeutet.

4. Substituierte 3,4-Dihydronaphthaline nach einem oder mehreren der Ansprüche 1 bis 3, dadurch gekennzeichnet, daß R¹ die Aminogruppe, R² ein Wasserstoffatom und R³ den Methylrest bedeutet.

5. Arzneimittel, dadurch gekennzeichnet, daß es als Wirkstoff wenigstens ein substituiertes 3,4-Dihydronaphthalin gemäß den Ansprüchen 1 bis 4 enthält und zur parenteralen, oralen, rektalen, topischen oder intranasalen Applikation geeignet ist.

6. Arzneimittel nach Anspruch 5, dadurch gekennzeichnet, daß es als Wirkstoff pro Einzeldosis 0,01 bis 1000 mg wenigstens eines substituierten 3,4-Dihydronapthalins gemäß den Ansprüchen 1 bis 4 enthält.

7. Arzneimittel nach einem oder beiden der Ansprüche 5 bis 6, dadurch gekennzeichnet, daß es zur parenteralen Applikation geeignet ist und als Wirkstoff pro Einzeldosis 0,1 bis 1000 mg wenigstens eines substituierten 3,4-Dihydronaphthalins gemäß den Ansprüchen 1 bis 4 enthält.

8. Arzneimittel nach einem oder beiden der Ansprüche 5 bis 6, dadurch gekennzeichnet, daß es in Form von Tabletten, Dragees oder Kapseln, gegebenenfalls mit verzögerter Wirkstofffreigabe, vorliegt und als Wirkstoff pro Einzeldosis 0,1 bis 1000 mg wenigstens eines substituierten 3,4-Dihydronaphthalins gemäß den Ansprüchen 1 bis 4 enthält.

9. Arzneimittel nach Anspruch 5 zur intranasalen oder oralen Verabreichung wenigstens eines substituierten 3,4-Dihydronaphthalins gemäß den Ansprüchen 1 bis 4 in Sprayform.

10. Verfahren zur Herstellung eines substituierten 3,4-Dihydronapthalins der allgemeinen Formel I 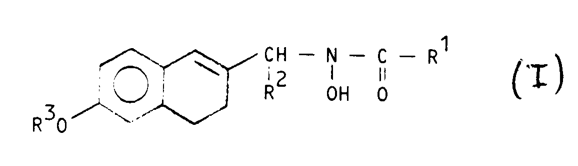 in der R¹ eine Methyl- oder Aminogruppe ist, R² ein Wasserstoffatom oder eine Methylgruppe darstellt und R³ einen geradkettigen oder verzweigten Alkylrest mit 1 bis 4 Kohlenstoffatomen, den Allyl- oder den Crotylrest oder eine Cyclopentylgruppe bedeutet, dadurch gekennzeichnet, daß man eine Verbindung der Formel II 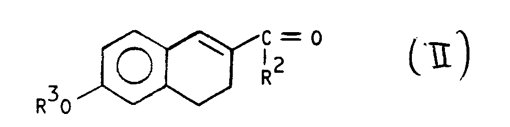 mit Hydroxylamin oder einem von dessen Salzen in Gegenwart von Basen zum Oxim umsetzt, dieses mit einem borhaltigen Reduktionsmittel in Gegenwart von Säuren zu einem Hydroxylamin der Formel III 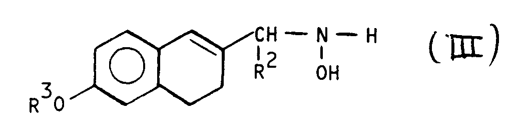 reduziert und in dieses anschließend den Rest -CO-R¹ einführt.

11. Verfahren nach Anspruch 10 dadurch gekennzeichnet, daß man ein Hydroxylamin der Formel III entweder
a) mit Trimethylsilylisocyanat umsetzt und anschließend die Trimethylsilylgruppe abspaltet,
oder
b) mit Kalium- oder Natriumcyanat in saurer Lösung oder mit Phosgen oder einem Chlorameisensäure(niedrig-alkyl)- oder - benzylester in Gegenwart eines säurebindenden Mittels und anschließend mit Ammoniak oder einer Ammoniak liefernden Verbindung umsetzt.

12. Verfahren nach Anspruch 10, dadurch gekennzeichnet, daß man ein Hydroxylamin der Formel III mit einem Acetylierungsmittel, vorzugsweise Acetanhydrid oder Acetylchlorid, in Gegenwart säurebindender Stoffe umsetzt und aus der so erhaltenen Verbindung der Formel IV 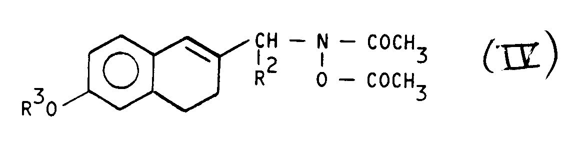 mit einer Base die O-Acetylgruppe abspaltet.

13. Verfahren nach Anspruch 10, dadurch gekennzeichnet, daß man das Oxim von einer Verbindung der Formel II mit einem Borhydrid, vorzugsweise mit Natriumcyanoborhydrid in Gegenwart von Säuren bei Temperaturen zwischen 20 und 60° C zu einem Hydroxylamin der Formel III reduziert.

14. Verfahren nach Anspruch 10, dadurch gekennzeichnet, daß man das Oxim von einer Verbindung der Formel II mit einem Boran-Amin-Komplex oder mit Boran-Tetrahydrofuran-Komplex in Gegenwart von Säuren bei Temperaturen zwischen 0 und 50° C zu einem Hydroxylamin der Formel III reduziert.

## Claims

1. Substituted 3,4-dihydronaphthalenes of the general formula I 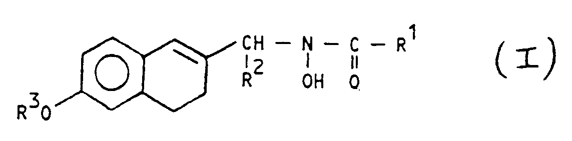 in which R¹ is a methyl or amino group, R² denotes a hydrogen atom or a methyl group and R³ means an unbranched or branched alkyl residue with 1 to 4 carbon atoms, an allyl or crotyl residue or a cyclopentyl group.

2. Substituted 3,4-dihydronaphthalenes according to claim 1, characterised in that R³ means a methyl residue.

3. Substituted 3,4-dihydronaphthalenes according to one or both of claims 1 to 2, characterised in that R¹ means an amino group.

4. Substituted 3,4-dihydronaphthalenes according to one or more of claims 1 to 3, characterised in that R¹ means an amino group, R² means a hydrogen atom and R³ means a methyl residue.

5. Pharmaceutical preparation, characterised in that it contains at least one substituted 3,4-dihydronaphthalene according to claims 1 to 4 as active constituent and is suitable for parenteral, oral, rectal, topical or intranasal administration.

6. Pharmaceutical preparation according to claim 5, characterised in that it contains as active constituent in each individual dose 0.01 to 1000 mg of at least one substituted 3,4-dihydronaphthalene according to claims 1 to 4.

7. Pharmaceutical preparation according to one or both of claims 5 to 6, characterised in that it is suitable for parenteral administration and contains as active constituent in each individual dose 0.1 to 1000 mg of at least one substituted 3,4-dihydronaphthalene according to claims 1 to 4.

8. Pharmaceutical preparation according to one or both of claims 5 to 6, characterised in that it is in the form of tablets, coated pills or capsules, optionally with controlled active constituent release and contains as active constituent in each individual dose 0.1 to 1000 mg of at least one substituted 3,4-dihydronaphthalene according to claims 1 to 4.

9. Pharmaceutical preparation according to claim 5 for intranasal or oral administration of at least one substituted 3,4-dihydronaphthalene according to claims 1 to 4 in spray form.

10. Process for the production of a substituted 3,4-dihydronaphthalene of the general formula I 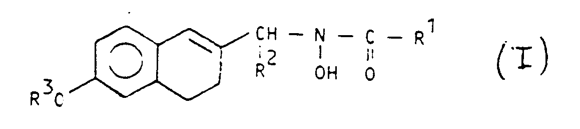 in which R¹ is a methyl or amino group, R² denotes a hydrogen atom or a methyl group and R³ means an unbranched or branched alkyl residue with 1 to 4 carbon atoms, an allyl or crotyl residue or a cyclopentyl group, characterised in that a compound of the formula II 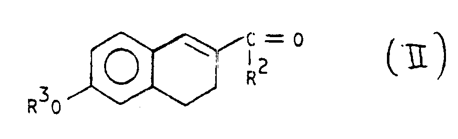 is reacted with hydroxylamine or a salt thereof in the presence of bases to yield the oxime, which is reduced with a reducing agent containing boron in the presence of acids to yield a hydroxylamine of the formula III 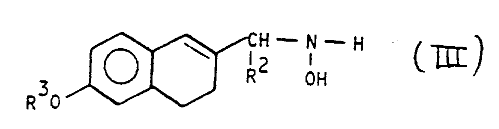 and the residue -CO-R¹ is then introduced into this compound.

11. Process according to claim 10, characterised in that a hydroxylamine of the formula III is either
a) reacted with trimethylsilyl isocyanate and the trimethylsilyl group then eliminated,
or
b) reacted with potassium cyanate or sodium cyanate in an acidic solution or with phosgene or a chloroformic (lower alkyl) ester or benzyl ester in the presence of an acid-binding agent and then reacted with ammonia or an ammonia-liberating compound.

12. Process according to claim 10, characterised in that a hydroxylamine of the formula III is reacted with an acetylating agent, preferably acetic anhydride or acetyl chloride, in the presence of acid-binding substances, and the O-acetyl group is eliminated from the resultant compound of the formula IV with a base

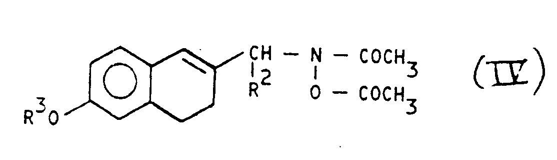

13. Process according to claim 10, characterised in that the oxime of a compound of the formula II is reduced with a borohydride, preferably with sodium cyanoborohydride in the presence of acids at temperatures of between 20 and 60°C to yield a hydroxylamine of the formula III.

14. Process according to claim 10, characterised in that the oxime of a compound of the formula II is reduced with a borane-amine complex or with borane-tetrahydrofuran complex in the presence of acids at temperatures of between 0 and 50°C to yield a hydroxylamine of the formula III.

## Revendications

1. 3,4-dihydronaphtalènes substitués de formule générale I 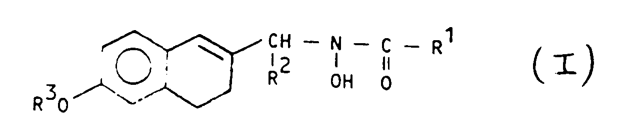 dans laquelle R¹ est un groupe méthyle ou un groupe amino, R² est un atome d'hydrogène ou un groupe méthyle et R³ est un reste alkyle linéaire ou ramifié ayant 1 à 4 atomes de carbone, le reste allyle ou le reste crotyle ou un groupe cyclopentyle.

2. 3,4-dihydronaphtalènes substitués suivant la revendication 1, caractérisés en ce que R³ représente le reste méthyle.

3. 3,4-dihydronaphtalènes substitués suivant l'une des revendications 1 et 2 ou les deux, caractérisés en ce que R¹ est un groupe amino.

4. 3,4-dihydronaphtalènes substitués suivant une ou plusieurs des revendications 1 à 3, caractérisés en ce que R¹ est le groupe amino, R² est un atome d'hydrogène et R³ est le reste méthyle.

5. Médicament, caractérisé en ce qu'il contient comme substance active au moins un 3,4-dihydronaphtalène substitué suivant les revendications 1 à 4 et convient pour l'administration parentérale, orale, rectale, topique ou intranasale.

6. Médicament suivant la revendication 5, caractérisé en ce qu'il contient comme substance active, par dose individuelle 0,01 à 1000 mg d'au moins un 3,4-dihydronaphtalène substitué suivant les revendications 1 à 4.

7. Médicament suivant l'une des revendications 5 et 6 ou les deux, caractérisé en ce qu'il convient pour l'administration parentérale et contient comme substance active, par dose individuelle, 0,1 à 1000 mg d'au moins un 3,4-dihydronaphtalène substitué suivant les revendications 1 à 4.

8. Médicament suivant l'une des revendications 5 et 6 ou les deux, caractérisé en ce qu'il se présente sous forme de comprimés, de dragées ou de gélules, le cas échéant avec libération retardée de la substance active, et contient comme substance active, par dose individuelle, 0,1 à 1000 mg d'au moins un 3,4-dihydronaphtalène substitué suivant les revendications 1 à 4.

9. Médicament suivant a revendication 5, destiné à l'administration intranasale ou orale d'au moins un 3,4-dihydronaphtalène substitué suivant les revendications 1 à 4, sous une forme pulvérisable.

10. Procédé de production d'un 3,4-dihydronaphtalène substitué de formule générale I 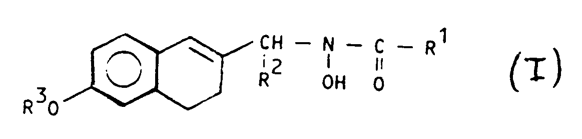 dans laquelle R¹ est un groupe méthyle ou un groupe amino, R² est un atome d'hydrogène ou un groupe méthyle et R³ est un reste alkyle linéaire ou ramifié ayant 1 à 4 atomes de carbone, le reste allyle ou le reste crotyle ou un groupe cyclopentyle,
caractérisé en ce qu'on fait réagir un composé de formule II 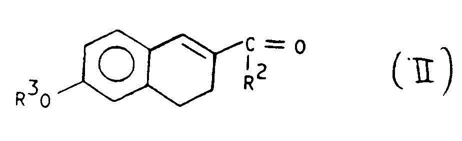 avec l'hydroxylamine ou l'un de ses sels en présence de bases pour former l'oxime, on réduit ce dernier avec un agent réducteur contenant du bore en présence d'acides en une hydroxylamine de formule III 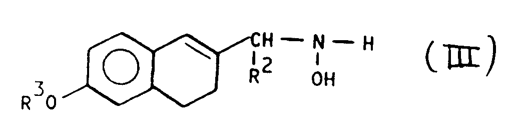 et on introduit dans cette dernière le reste -CO-R¹.

11. Procédé suivant la revendication 10, caractérisé en ce qu'on fait réagir une hydroxylamine de formule III
a) avec l'isocyanate de triméthylsilyle puis on élimine le groupe triméthylsilyle
ou bien
b) avec le cyanate de potassium ou de sodium en solution acide ou avec le phosgène et un ester d'alkyle inférieur ou de benzyle de l'acide chloroformique en présence d'un accepteur d'acide puis on fait réagir le produit avec l'ammoniac ou un composé libérant de l'ammoniac.

12. Procédé suivant la revendication 10, caractérisé en ce qu'on fait réagir l'hydroxylamine de formule III avec un agent d'acétylation, de préférence l'acétanhydride ou le chlorure d'acétyle, en présence d'accepteurs d'acides et on élimine le groupe O-acétyle avec une base du composé ainsi obtenu de formule IV

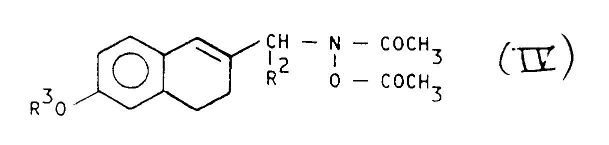

13. Procédé suivant la revendication 10, caractérisé en ce qu'on réduit l'oxime d'un composé de formule II avec un borohydrure, de préférence avec le cyanoborohydrure de sodium en présence d'acides, à des températures comprises entre 20 et 60°C pour obtenir une hydroxylamine de formule III.

14. Procédé suivant la revendication 10, caractérisé en ce qu'on réduit l'oxime d'un composé de formule II avec un complexe borane-amine ou un complexe de borane-tétrahydrofuranne en présence d'acides à des températures comprises entre 0 et 50°C pour obtenir une hydroxylamine de formule III.
